# EUROPEAN PATENT SPECIFICATION

(11) **EP 3 817 659 B1**
(45) Date of publication and mention of the grant of the patent: **05.06.2024**
(21) Application number: 19745843.3
(22) Date of filing: 02.07.2019
(51) Int. Cl.: A61B 5/06

(54) **MAGNETIC TRACKING TRANSMITTER**
MAGNETISCHER SPURFOLGESENDER
TRANSMETTEUR DE REPÉRAGE MAGNÉTIQUE

(30) Priority: 02.07.2018 US 201862693433 P
(43) Date of publication of application: 12.05.2021
(73) Proprietor: Boston Scientific Scimed Inc., Maple Grove, Minnesota 55311 (US)
(72) Inventor: PLOTKIN, Anton, Plymouth, Minnesota 55446 (US); FOSTER, Daniel J., Lino Lakes, Minnesota 55038 (US); MORRIS, Eric, Knoxville, Tennessee 37922 (US); CHIZEK, David A., Brooklyn Park, Minnesota 55443 (US); BERNHOLT, Peg, White Bear Lake, Minnesota 55110 (US); SULLIVAN, Timothy, La Mesa, California 91942 (US); MUSTO, Peter J., Woburn, Massachusetts 01801 (US)
(74) Representative: Pfenning, Meinig & Partner mbB
(86) International application number: PCT/US2019/040269
(87) International publication number: WO 2020/010063

(56) References cited:
- WO-A2-2018/109555
- US-A- 3 448 421
- US-A1- 2010 137 705
- US-A1- 2011 224 537
- US-A1- 2015 216 490

## Description

### TECHNICAL FIELD

The present disclosure relates to systems, methods, and devices for tracking items. More specifically, the disclosure relates to systems, methods, and devices for electro-magnetically tracking medical devices used in medical procedures.

### BACKGROUND

A variety of systems, methods, and devices can be used to track medical devices. Tracking systems can use generated magnetic fields that are sensed by at least one tracking sensor in the tracked medical device. The generated magnetic fields provide a fixed frame of reference, and the tracking sensor senses the magnetic fields to determine the location and orientation of the sensor in relation to the fixed frame of reference.

Document WO 2018/109555 A2 relates to a magnetic field generator comprising a plurality of magnetic field transmitters, each comprising interlacing layers of conductive material, configured to provide increased magnetic strength and minimal fluoroscopic occlusion.

Document US 3 448 421 A relates to means for eddy current shielding of small magnetic cores which is easy to fabricate and which reduces the available winding space by a negligible degree.

### SUMMARY

The invention is as specified in the appended claims.

While multiple embodiments are disclosed, still other embodiments of the present disclosure will become apparent to those skilled in the art from the following detailed description, which shows and describes illustrative embodiments of the disclosure. Accordingly, the drawings and detailed description are to be regarded as illustrative in nature and not restrictive.

### BRIEF DESCRIPTION OF THE DRAWINGS

FIG. 1 shows a schematic of a tracking system, in accordance with certain embodiments of the present disclosure.
FIG. 2 shows a block representation of a computing device, in accordance with certain embodiments of the present disclosure.
FIG. 3 shows a perspective view of a magnetic field transmitter assembly, in accordance with certain embodiments of the present disclosure.
FIG. 4 shows a top view of the magnetic field transmitter assembly of FIG. 3, in accordance with certain embodiments of the present disclosure.
Figures 5A and 5B show schematics of a magnetic field generator assembly, in accordance with certain embodiments of the present disclosure.
FIG. 6 shows a partial, cross-sectional view of an exterior of the magnetic field transmitter assembly of Figures 3 and 4, in accordance with certain embodiments of the present disclosure.
FIG. 7 shows a schematic top view of a woven layer of the magnetic field transmitter assembly of Figures 3 and 4, in accordance with certain embodiments of the present disclosure.
Figures 8A-8C show various arrangements of a magnetic field transmitter assembly, in accordance with certain embodiments of the present disclosure.
Figures 9-11 show various magnetic field transmitter assemblies, in accordance with certain embodiments of the present disclosure.

While the invention is amenable to various modifications and alternative forms, specific embodiments have been shown by way of example in the drawings and are described in detail below.

### DETAILED DESCRIPTION

During medical procedures, medical devices such as probes (e.g., catheter, imaging probe, diagnostic probe) are inserted into a patient. To track the location and orientation of a probe within the patient, probes can be provisioned with magnetic field sensors that detect various magnetic fields generated by magnetic field generators near the patient. The amplitude and/or phase of the detected magnetic fields can be used to determine location and orientation of the probe. Certain embodiments of the present disclosure are accordingly directed to systems, methods, and devices including magnetic field transmitter assemblies that generate magnetic fields.

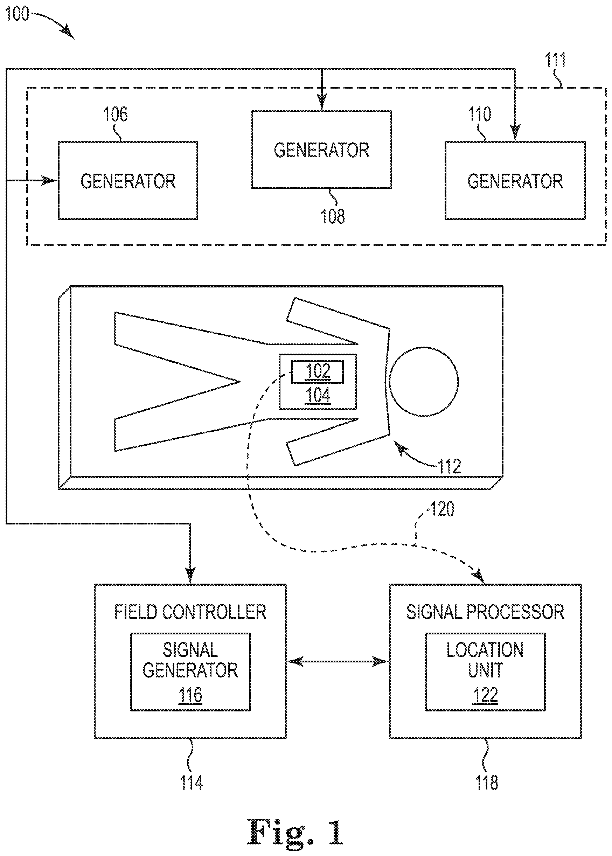

FIG. 1 is a schematic block diagram depicting a tracking system 100 that is configured to determine location information corresponding to the medical device 104 based on information collected using a receiver (e.g., sensor) 102 associated with a medical device 104 (e.g., probe). The information collected by the receiver 102 includes a received field signal corresponding to an electromagnetic field defined by a set of electromagnetic signals transmitted by one or more magnetic field generator assemblies 106, 108, and 110. Although only three magnetic field generator assemblies are shown, the system 100 can include fewer or more magnetic field generator assemblies, as will be described in more detail below. For example, to provide six-degree-of-freedom tracking, the tracking system 100 should include at least one magnetic field generator assembly when the receiver 102 includes a three-axis sensor (e.g., three-axis magnetic sensor). Additional magnetic field generator assemblies can be used to extend the range and accuracy of tracking. When the receiver 102 includes a dual-axis sensor (e.g., dual-axis magnetic sensor), the tracking system 100 should include at least two magnetic field generator assemblies. In embodiments with multiple magnetic field generator assemblies, the magnetic field generator assemblies can be coupled to a common housing (together, the magnetic field generator assemblies, the housing, and other components forming a magnetic field transmitter assembly) or placed individually. The magnetic field transmitter assembly can be placed under a patient's bed, under the patient but above the patient's bed, and/or placed above the patient (e.g., placed directly on top of the patient or suspended above the patient).

The magnetic field generator assemblies may be coil-based (e.g., includes one or more coil windings), and/or permanent-magnet-based-each of which is discussed in more detail below. According to embodiments, one or more magnetic field generator assemblies 106, 108, and 110, are configured to transmit (e.g., radiate) electromagnetic signals, which produce a magnetic field within which a subject 112 is disposed. In FIG. 1, the magnetic field generator assemblies 106, 108, and 110 are positioned within a magnetic field transmitter assembly 111. According to embodiments, the system 100 includes a magnetic field controller 114 configured to manage operation of the magnetic field generator assemblies 106, 108, and 110.

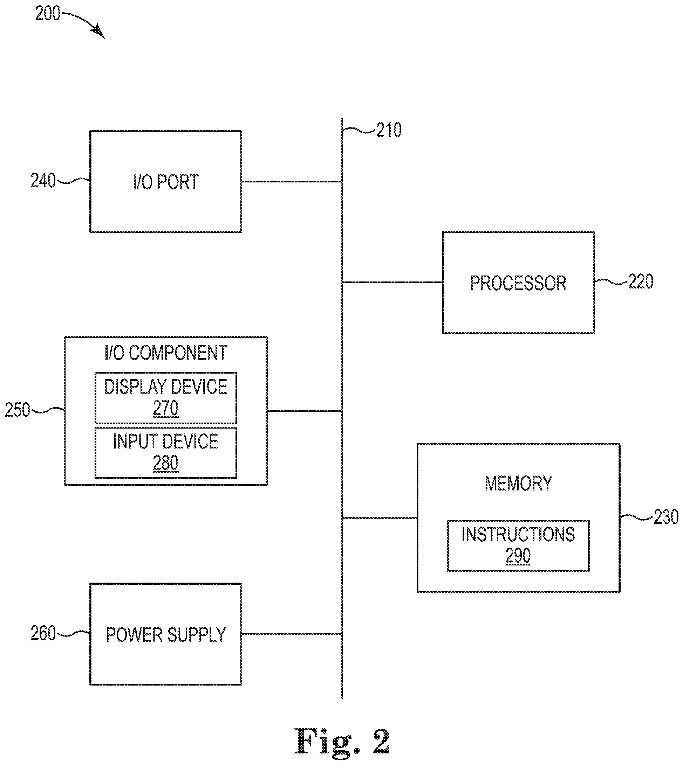

As shown in FIG. 1, the magnetic field controller 114 includes a signal generator 116 configured to provide driving current to each of the magnetic field generator assemblies 106, 108, and 110, causing each magnetic field generator assembly to transmit one or more electromagnetic signals (e.g., magnetic fields). In certain embodiments, the signal generator 116 is configured to provide sinusoidal driving currents to the magnetic field generator assemblies 106, 108, and 110. The magnetic field controller 114 can be implemented using firmware, integrated circuits, and/or software modules that interact with each other or are combined together. For example, the magnetic field controller 114 may include computer-readable instructions/code for execution by a processor (see FIG. 2). Such instructions may be stored on a non-transitory computer-readable medium (see FIG. 2) and transferred to the processor for execution. In some embodiments, the magnetic field controller 114 can be implemented in one or more application-specific integrated circuits and/or other forms of circuitry suitable for controlling and processing magnetic tracking signals and information.

The receiver 102 (e.g., magnetic field sensor) (which may include one or more receivers/sensors) may be configured to produce an electrical response to the magnetic field(s) generated by the magnetic field generator assemblies 106, 108, and 110. For example, the receiver 102 may include a magnetic field sensor such as inductive sensing coils and/or various sensing elements such as magneto-resistive (MR) sensing elements (e.g., anisotropic magneto-resistive (AMR) sensing elements, giant magneto-resistive (GMR) sensing elements, tunneling magneto-resistive (TMR) sensing elements, Hall effect sensing elements, colossal magneto-resistive (CMR) sensing elements, extraordinary magneto-resistive (EMR) sensing elements, spin Hall sensing elements, and the like), giant magneto-impedance (GMI) sensing elements, and/or flux-gate sensing elements.

The sensed magnetic field signal may include multiple magnetic field signals, each of which may be processed to extract field components corresponding to one or more magnetic field generator assemblies. The sensed magnetic field signal is communicated to a signal processor 118, which is configured to analyze the sensed magnetic field signal to determine location information corresponding to the receiver 102 (and, thus, the medical device 104). Location information may include any type of information associated with a location and/or position of a medical device 104 such as, for example, location, relative location (e.g., location relative to another device and/or location), position, orientation, velocity, acceleration, and/or the like. As mentioned above, the tracking system 100 can utilize amplitude and/or phase (e.g., differences in phase) of the sensed magnetic field signal to determine location and orientation of the probe.

The tracking system 100 can also include at least one sensor that is configured and arranged to sense the magnetic fields generated by the magnetic field generator assemblies, 106-110. The sensor can be a magnetic sensor (e.g., dual-axis magnetic sensor, tri-axis magnetic sensor) and be positioned at a known reference point in proximity to the magnetic field generator assemblies, 106-110, to act as a reference sensor. For example, one or more sensors can be coupled to the subject's bed, an arm of an x-ray machine, or at other points a known distance from the magnetic field generator assemblies, 106-110. In some embodiments, the at least one sensor is mounted to one of the magnetic field generator assemblies, 106-110.

The medical device 104 may include, for example, a catheter (e.g., a mapping catheter, an ablation catheter, a diagnostic catheter, an introducer), an endoscopic probe or cannula, an implantable medical device (e.g., a control device, a monitoring device, a pacemaker, an implantable cardioverter defibrillator (ICD), a cardiac resynchronization therapy (CRT) device, a CRT-D), guidewire, endoscope, biopsy needle, ultrasound device, reference patch, robot and/or the like. For example, in embodiments, the medical device 104 may include a mapping catheter associated with an anatomical mapping system. The medical device 104 may include any other type of device configured to be at least temporarily disposed within a subject 112. The subject 112 may be a human, a dog, a pig, and/or any other animal having physiological parameters that can be recorded. For example, in embodiments, the subject 112 may be a human patient.

As shown in FIG. 1, the medical device 104 may be configured to be disposed within the body of a subject 112, and may be configured to be communicatively coupled to the signal processor 118 via a communication link 120 (shown in phantom). In embodiments, the communication link 120 may be, or include, a wired communication link (e.g., a serial communication), a wireless communication link such as, for example, a short-range radio link, such as Bluetooth, IEEE 802.11, a proprietary wireless protocol, and/or the like. The term "communication link" may refer to an ability to communicate some type of information in at least one direction between at least two devices, and should not be understood to be limited to a direct, persistent, or otherwise limited communication channel. That is, in some embodiments, the communication link 120 may be a persistent communication link, an intermittent communication link, an ad-hoc communication link, and/or the like. The communication link 120 may refer to direct communications between the medical device 104 and the signal processor 118, and/or indirect communications that travel between the medical device 104 and the signal processor 118 via at least one other device (e.g., a repeater, router, hub, and/or the like). The communication link 120 may facilitate uni-directional and/or bi-directional communication between the medical device 104 and the signal processor 118. Data and/or control signals may be transmitted between the medical device 104 and the signal processor 118 to coordinate the functions of the medical device 104 and/or the signal processor 118.

The signal processor 118 further includes a location unit 122 configured to determine, based on the sensed field signal (e.g., the phase, amplitude, differences in phase and/or amplitude of the sensed field signal), location information corresponding to the medical device 104. The location unit 122 may be configured to determine location information according to any location-determination technique that uses magnetic navigation. According to various embodiments of the disclosed subject matter, any number of the components depicted in FIG. 1 (e.g., the field controller 114, the signal generator 116, the signal processor 118) may be implemented on one or more computing devices, either as a single unit or a combination of multiple devices. The system 100 can include a display for visualizing the position and/or orientation of the medical device 104 in the subject 112.

FIG. 2 is a schematic block diagram depicting an illustrative computing device 200, in accordance with embodiments of the disclosure. The computing device 200 may include any type of computing device suitable for implementing aspects of embodiments of the disclosed subject matter. Examples of computing devices include specialized computing devices or general-purpose computing devices such "workstations," "servers," "laptops," "desktops," "tablet computers," "hand-held devices," "general-purpose graphics processing units (GPGPUs)," and the like, all of which are contemplated within the scope of Figures 1 and 2, with reference to various components of the tracking system 100 and/or computing device 200.

In embodiments, the computing device 200 includes a bus 210 that, directly and/or indirectly, couples the following devices: a processor 220, a memory 230, an input/output (I/O) port 240, an I/O component 250, and a power supply 260. Any number of additional components, different components, and/or combinations of components may also be included in the computing device 200. The I/O component 250 may include a presentation component configured to present information to a user such as, for example, a display device, a speaker, a printing device, and/or the like, and/or an input component such as, for example, a microphone, a joystick, a satellite dish, a scanner, a printer, a wireless device, a keyboard, a pen, a voice input device, a touch input device, a touch-screen device, an interactive display device, a mouse, and/or the like.

The bus 210 represents what may be one or more busses (such as, for example, an address bus, data bus, or combination thereof). Similarly, in embodiments, the computing device 200 may include a number of processors 220, a number of memory components 230, a number of I/O ports 240, a number of I/O components 250, and/or a number of power supplies 260. Additionally any number of these components, or combinations thereof, may be distributed and/or duplicated across a number of computing devices. As an example only, the processor 220 may include the signal processor 118, but other suitable configurations are also contemplated to suit different applications.

In embodiments, the memory 230 includes computer-readable media in the form of volatile and/or nonvolatile memory and may be removable, nonremovable, or a combination thereof. Media examples include Random Access Memory (RAM); Read Only Memory (ROM); Electronically Erasable Programmable Read Only Memory (EEPROM); flash memory; optical or holographic media; magnetic cassettes, magnetic tape, magnetic disk storage or other magnetic storage devices; data transmissions; and/or any other medium that can be used to store information and can be accessed by a computing device such as, for example, quantum state memory, and/or the like. In embodiments, the memory 230 stores computer-executable instructions 290 for causing the processor 220 to implement aspects of embodiments of system components discussed herein and/or to perform aspects of embodiments of methods and procedures discussed herein.

The computer-executable instructions 290 may include, for example, computer code, machine-useable instructions, and the like such as, for example, program components capable of being executed by one or more processors 220 associated with the computing device 200. Program components may be programmed using any number of different programming environments, including various languages, development kits, frameworks, and/or the like. Some or all of the functionality contemplated herein may also, or alternatively, be implemented in hardware and/or firmware.

The illustrative computing device 200 shown in FIG. 2 is not intended to suggest any limitation as to the scope of use or functionality of embodiments of the present disclosure. Neither should the illustrative computing device 200 be interpreted as having any dependency or requirement related to any single component or combination of components illustrated therein. Additionally, various components depicted in FIG. 2 may be, in embodiments, integrated with various ones of the other components depicted therein (and/or components not illustrated), all of which are considered to be within the ambit of the present disclosure.

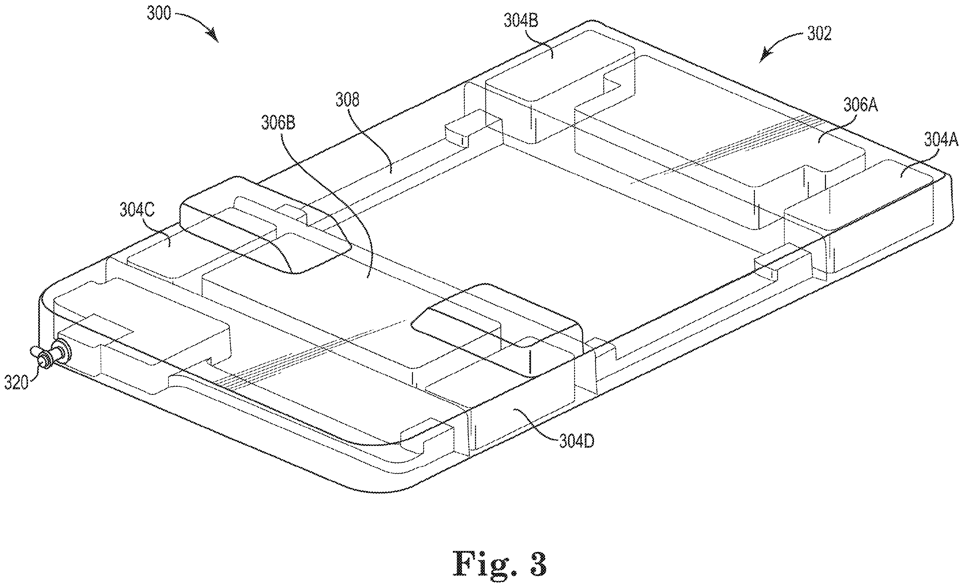

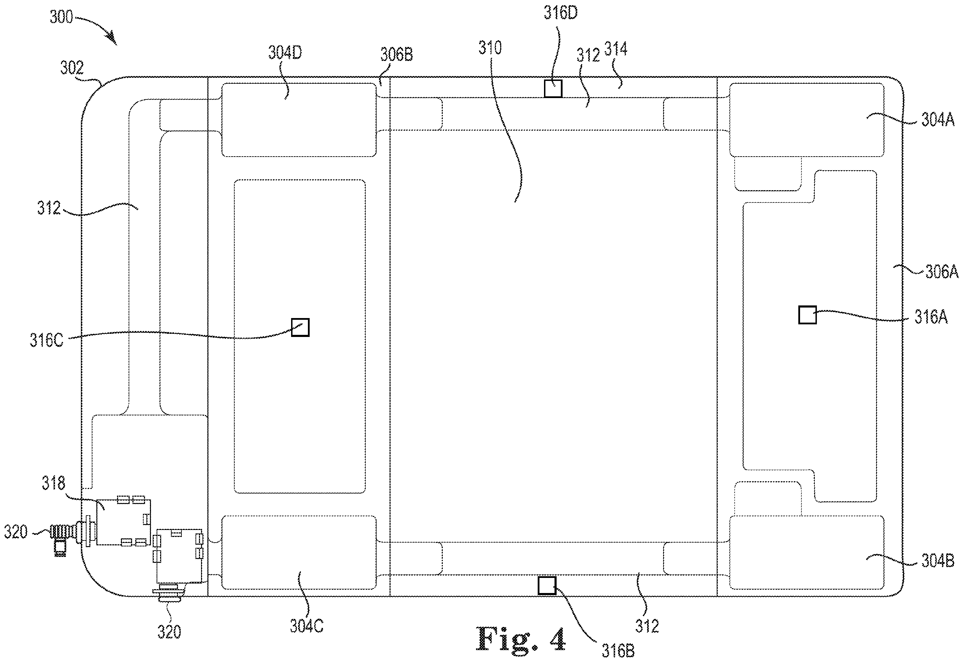

FIG. 3 shows an exploded view of a magnetic field transmitter assembly 300 including a housing 302 and a plurality of magnetic field generator assemblies (e.g., a first magnetic field generator assembly 304A, a second magnetic field generator assembly 304B, a third magnetic field generator assembly 304C, and a fourth magnetic field generator assembly 304D). FIG. 4 shows a top view of an interior of the magnetic field transmitter assembly 300. Although four magnetic field generator assemblies are shown in the figures, the magnetic field transmitter assembly 300 can include fewer or more magnetic field generator assemblies.

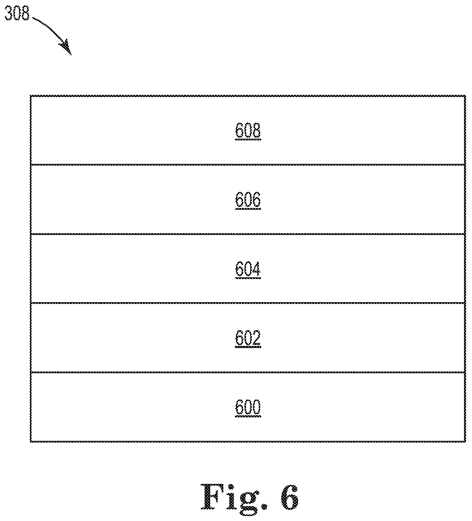

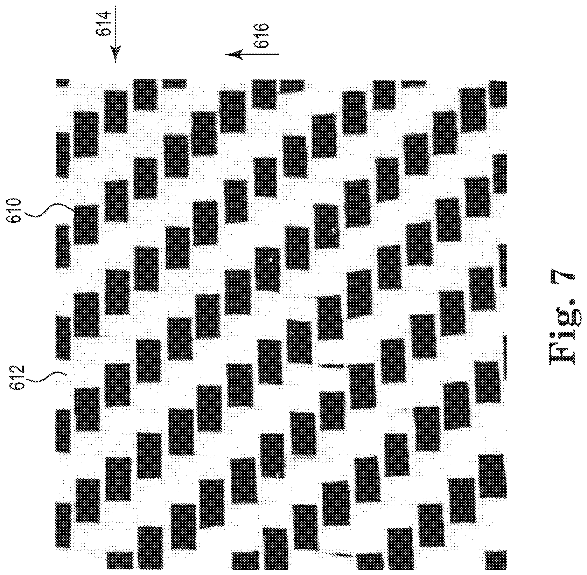

As shown in FIG. 4, the housing 302 includes at least one sub-assembly (e.g., 306A and 306B) in which one or more of the magnetic field generator assemblies 304A-D is positioned and fixed in the housing 302. The sub-assemblies, 306A and 306B, can comprise a rigid, low-to-non-conductive, and low-to-non-ferrous material such as fiber-filled composites (e.g., glass-, carbon-fiber-, and/or aramid-filled polyether ether ketone or polyetherimide) and can help maintain a relative position of the magnetic field generator assemblies 304A-D with respect to each other and other components (e.g., reference sensors) of the magnetic field transmitter assembly 300. FIG. 4 shows the magnetic field generator assemblies 304A-D are similarly aligned (e.g., aligned in the same direction) within the housing 302 and positioned with respect to each other in a rectangular shape (e.g., one magnetic field generator assembly at each corner of the rectangle) within the housing 302, although the magnetic field generator assemblies can be positioned in other shapes (e.g., square, polygonal). As shown in FIG. 3, the magnetic field generator assemblies 304A-D are cuboid shaped (e.g., at least one rectangular cross-section), but, as will be discussed in more detail below, the magnetic field generator assemblies 304A-D can form other shapes (e.g., cubes). In embodiments with multiple sub-assemblies, the sub-assemblies, 306A and 306B, can be directly or indirectly coupled to each other. The housing 302 also includes a multi-layer skin 308 (discussed in more detail below with respect to Figures 6 and 7) that forms an exterior shape of the housing 308. Space between the sub-assemblies, 306A and 306B, and the multi-layer skin 308 can be filled with a filler 310 such as a lightweight, machinable foam that adds some structural rigidity to the housing 302 and that is flouro-translucent to mitigate interference with x-rays. The filler 310 can include channels 312 through which electrical wiring between or among the magnetic field generator assemblies 304A-D can be routed within the magnetic field transmitter assembly 300. In certain embodiments, the channels 312 are arranged such that they extend through the filler 310 near a perimeter 314 of housing 302 so that the electrical wiring does not generate magnetic fields that interfere with the magnetic fields generated by the magnetic field generator assemblies 304A-D. The magnetic field transmitter assembly 300 may also include one or more reference sensors 316A-D (e.g., magnetic field sensor) positioned near (e.g., 2-3 cm) from each magnetic field generator assembly 304A-D. The reference sensors 316A-D are configured and positioned to detect the magnetic fields generated by respective magnetic field generator assemblies 304A-D. Sensor signals indicative of the detected magnetic fields can be sent to a magnetic field controller (e.g., the magnetic field controller 114 of FIG. 1) to assist with generating desired magnetic fields.

The magnetic field generator assemblies 304A-D can be electrically coupled to circuitry 318 (e.g., printed circuit boards and associated electrical components) positioned within the housing 302. The circuitry 318 can include memory for storing calibration data and circuitry that transmits electrical signals from a magnetic field controller (e.g., the magnetic field controller 114 of FIG. 1) and/or a signal generator (e.g., the signal generator 116 of FIG. 1) to power and control the magnetic field generator assemblies 304A-D. As such, the housing 302 may have one or more electrical connectors 320 positioned within an opening in the housing 302 to transmit electrical signals to and from the magnetic field transmitter assembly 300. The circuitry 318 can be positioned near the perimeter 314 of the housing 302 to mitigate the risk of the circuitry 318 generating magnetic fields that interfere with the magnetic fields generated by the magnetic field generator assemblies 304A-D.

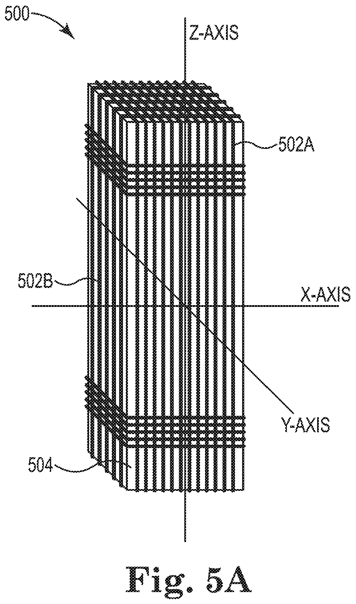

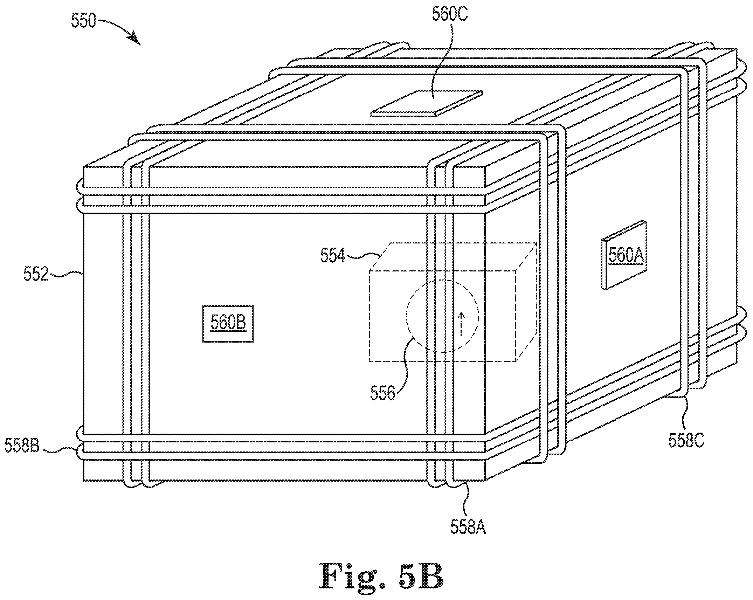

Figures 5A and 5B show schematics of magnetic field generator assemblies which can be used in the tracking system 100 and positioned within a magnetic field transmitter assembly (e.g., the magnetic field transmitter assembly 300 of FIG. 3). The magnetic field generator assembly in FIG. 5A is coil-based and the magnetic field generator assembly of FIG. 5B is coil- and permanent-magnet-based. FIG. 5A shows a magnetic field generator assembly 500 that includes first coil windings 502A, second coil windings 502B, and third coil windings 502C, which can comprise conductive materials (e.g., copper). For reference, FIG. 5A features an x-axis, a y-axis, and a z-axis. The first coil windings 502A are wrapped around the x-axis and around a core 504. The core 504 can be one of an air core, ferromagnetic material core, and antiferromagnetic material core. As shown, the core 504 is cuboid-shaped. The second coil windings 502B are wrapped around the y-axis and the core 504, and the third coil windings 502C are wrapped around the z-axis and the core 504.

In certain embodiments, the first coil windings 502A, the second coil windings 502B, and the third coil windings 502C are layered such that some coil windings overlap others. Although FIG. 5A shows spacing between windings of the coil windings, the first, second, and third coil windings 502A-C can be wound such that the external faces of the coil windings form a wall-like structure comprising windings with little to no spacing between the windings.

As shown in FIG. 5A, the first coil windings 502A, the second coil windings 502B, and the third coil windings 502C are positioned in an orthogonal arrangement such that the first coil windings 502A, the second coil windings 502B, and the third coil windings 502C are wound around axes (e.g., x-axis, y-axis, and z-axis) that are substantially orthogonal to each other. Such an arrangement of coil windings can be considered to be a tri-axial orthogonal coil arrangement. As will be discussed in more detail below, when energized (e.g., when current is passed through the coil windings), the magnetic field generator assembly 500 generates a magnetic field.

FIG. 5B shows a schematic of a magnetic field generator assembly 550 for generating a rotating magnetic field. The magnetic field generator assembly 550 includes an enclosure 552, a subassembly 554 (in dotted lines), a magnet 556 (in dotted lines), a first set of coils 558A, a second set of coils 558B, a third set of coils 558C, a first magnetic field sensor 560A, a second magnetic field sensor 560B, and a third magnetic field sensor 560C. Each set of coils can be comprised of a single wire with multiple coil turns around the enclosure 552. The coils can be comprised of conductive materials such as copper. When energized, the first set of coils 558A, the second set of coils 558B, and the third set of coils 558C generate magnetic fields that can be controlled to rotate the permanent magnet 556 within the subassembly 554.

The first set of coils 558A, the second set of coils 558B, and the third set of coils 558C are wrapped around the enclosure 552, which houses the subassembly 554 and the magnet 556. Although the enclosure 552 is shown as being cube shaped, the enclosure 552 can be other shapes (e.g., cuboid, sphere, ellipsoid, cylindrical). For simplicity, only a few coil turns are shown as being included in the first, second and third set of coils 558A-C, but each set of coils can include many coils turns. In some embodiments, the sets of coils are wrapped such that the coils substantially cover the enclosure 552. In some embodiments, the sets of coils are wrapped orthogonally with respect to each other. In some embodiments, each set of coils is wrapped in a Helmholtz coil arrangement, which can be referred to as a 3-axis Helmholtz arrangement. For example, as shown in FIG. 5B, each set of coils includes subsets of coil windings at ends (e.g., near vertices) of the enclosure 552 that are separated by an area without coil windings. Helmholtz coils may provide a more uniform magnetic field within a volume of the enclosure 552 compared to a magnetic field generated by sets of a single, continuous coils covering faces of the enclosure 552.

The magnet 556 can comprise various magnetic materials such as Nd, Fe, Co, Sm, and the like. For example, the magnet 556 can comprise permanent magnetic materials such as NdFeB, SmCo, and the like. In some embodiments, the magnet 556 includes a coating that is coated around the permanent magnetic materials and that has a low coefficient of friction and/or high resistance to wear. For example, the coating can include metal plating (e.g., Ni) surrounding the permanent magnetic materials. In certain embodiments, the magnet 556 is coated with a low-friction plastic material such as polyoxymethylene or polytetrafluoroethylene. The magnet 556 can be a dipole, have a preferred magnetic orientation (represented by an arrow), and have a uniform magnetization throughout the permanent magnetic material within the magnet 556. In some embodiments, the magnet 366 is sphere shaped and has a diameter of 0.25" to 2". In some embodiments, the magnet 366 has a diameter of 0.25" to 1".

The magnet 556 can be positioned in the subassembly 554, which can form a clam-shell-like enclosure around the magnet 556. The subassembly 554 and the enclosure 552 can comprise non-magnetic materials with low coefficients of friction (e.g., polytetrafluoroethylenes, ceramics). In certain embodiments, the subassembly 554 comprises materials having a low coefficient of friction with respect to the coating of the magnet 556. A lubricant (e.g., oils, waxes, silicone, graphite, fluoropolymers) can be applied to internal surface of the subassembly 554 to reduce friction between the internal surfaces and the magnet 556. During operation, the magnetic field generator assembly 550 may be configured to generate a rotating magnetic field. The rotating magnetic field is generated by rotating the magnet 556. The magnet 556 is rotated by applying and controlling current applied to the coils surrounding the enclosure 552. The applied current generates a magnetic field that, when controlled, causes the magnet 556 to rotate via a controlled torque. The first magnetic field sensor 560A, the second magnetic field sensor 560B, and the third magnetic field sensor 560C can be coupled to the enclosure 552 and be configured to sense the rotating magnetic field generated by the magnet 556. The first, second, and third magnetic field sensor 560A-C can include inductive sensing coils and/or various sensing elements such as MR sensing elements, GMI sensing elements, and/or flux-gate sensing elements.

Referring back to Figures 3-4, the magnetic field generator assemblies 304A-D can include magnetic field generator assemblies like those shown in Figures 5A and 5B (i.e., magnetic field generator assemblies 500 and 550). As mentioned above, the magnetic field generator assemblies 304A-D are each configured to generate a magnetic field. In certain embodiments, the magnetic field generator assemblies 304A-D are configured to generate a rotating magnetic field. Rotating magnetic field-based tracking can utilize phase of the detected rotating magnetic fields to determine location and orientation of a probe (e.g., medical device 104 of FIG. 1).

When energized (e.g., when current is passed through the coil windings of the magnetic field generator assembly), each magnetic field generator assembly 304A-D is configured to generate a magnetic field. The magnetic fields are generated by applying current at particular frequencies and/or at particular time intervals. Using the magnetic field generator assembly 500 of FIG. 5A as an example, the magnetic field can be generated by applying current to the first coil windings 558A, the second coil windings 558B, and the third coil windings 558C.

In certain embodiments, each coil winding is energized sequential one at a time such that only one coil winding of all the windings in the magnetic field transmitter assembly 300 is energized at a given point in time. This sequential energizing can be considered to be a timing-based multiplexing approach. When using a timing-based multiplexing approach, each coil winding can be energized multiple times per second (e.g., 20 times/second, 40 times/second, 60 times/second). In certain embodiments, the current applied to each coil windings has a different frequency. The current frequencies can be 500 Hz to 10 kHz, 500 Hz to 30 kHz, for example, and can differ between magnetic field generator assemblies by approximately 25 Hz to 1 kHz (e.g., 50 Hz, 100 Hz, 500 Hz, and 1 kHz). For example, one coil winding may be energized by a current at a frequency of 800 Hz while another coil winding may be energized by current at 840 Hz while another coil winding may be energized by current at 880 Hz. In this example, the energizing currents differ in frequency by 40 Hz.

In certain embodiments, each coil winding of the magnetic field generator assemblies 304A-D is energized simultaneously but at different frequencies. For example, if each of the four magnetic field generator assemblies includes three sets of coil windings, then the magnetic field transmitter assembly would transmit magnetic fields at twelve different frequencies. Energizing the coil windings at different frequencies can be considered to be a frequency-based multiplexing approach. The current frequencies can be 500 Hz to 10 kHz, 500 Hz to 30 kHz, for example, and can differ between magnetic field transmitter assemblies by approximately 25 Hz to 1 kHz (e.g., 50 Hz, 100 Hz, 500 Hz, and 1 kHz). For example, one coil winding may be energized by a current at a frequency of 800 Hz while another coil winding may be energized by current at 840 Hz while another coil winding may be energized by current at 880 Hz. In this example, the energizing currents differ in frequency by 40 Hz.

The magnetic field generator assemblies 304A-D can be positioned such that their respective generated magnetic fields substantially fill and/or partially overlap each other in a shared magnetic field volume within and above the housing 302. In certain embodiments, the shared magnetic field volume is centrally located within the rectangular arrangement of the magnetic field generator assemblies 304A-D. The shared magnetic field volume should be positioned where the receiver/sensor (e.g., receiver 102 in FIG. 1) and to-be-tracked medical device (e.g., medical device 104 in FIG. 1) is be likely positioned during a procedure. For example, the arrangement of magnetic field generator assemblies 304A-D (via positioning of the magnetic field transmitter assembly 300) can be positioned such that the shared magnetic field volume covers a patient's heart and/or lung.

One concern with magnetic field transmitter assemblies is the structural rigidity of the magnetic field transmitter assemblies and their ability to maintain predefined dimensions between magnetic field generator assemblies, reference sensors, and other components. Another concern is the weight of magnetic field transmitter assemblies. Although materials such as carbon fiber can provide the type of structural rigidity and weight desired for magnetic field transmitter assemblies, carbon fiber can be electrically conductive. When subjected to a magnetic field (e.g., the magnetic fields generated by the magnetic field generator assemblies 304A-D), electrical conductors such as carbon fiber generate eddy currents, which, in turn, generate magnetic fields themselves. As such, carbon fiber can be a source of magnetic field distortion when used in magnetic field transmitter assemblies. Certain embodiments of the present disclosure accordingly are directed to housing materials and arrangements that help address the concerns listed above, including mitigating magnetic field distortion.

FIG. 6 shows a partial, cross-sectional view of the multi-layer skin 308. As mentioned above, the multi-layer skin provides an external shape of the housing 302 and therefore magnetic field transmitter assembly 300. The multi-layer skin 308 is shown as having a first layer 600, a second layer 602, a third layer 604, a fourth layer 606, and a fifth layer 608.

In certain embodiments, the first layer 600, the third layer 604, and the fifth layer 608 comprise an electrically-conductive material such as carbon fiber, which provides structural rigidity and which is lightweight. In certain embodiments, the first layer 600, the third layer 604, and the fifth layer 608 also comprise an electrically-insulating material such as para-aramid fibers. In such embodiments, the electrically-conductive material (e.g., carbon fibers) and the electrically-insulating material (e.g., para-aramid fibers) can be woven together to form individual layers. FIG. 7 shows a schematic top view of a layer woven with an electrically-conductive material 610 (in black) and an electrically-insulating material 612 (in white). Each material can be separated into tows, which can be woven into various patterns. In a first direction 614, the weave pattern shown in FIG. 7 includes tows of the electrically-conductive material 610 that each go under two tows of the electrically-insulating material 612 and then over two tows of the electrically-insulating material 612. In a second direction 616 (e.g., orthogonal to the first direction 614), each two of the electrically-insulating material 612 goes under two tows of the electrically-conductive material 610 and then over two tows of the electrically-conductive material 610. As indicated in FIG. 7, the electrically-conductive material 610 generally extends in a single direction (i.e., the first direction 614). It has been found that combining the electrically-conductive material and the electrically-insulating material helps reduce the bulk electrical conductivity of the layers containing an electrically-conductive material. In certain embodiments, the weave pattern of the third layer 604 is rotated approximately ninety degrees from the same weave patterns in the first layer 600 and the fifth layer 608. This alternating layer-by-layer alignment can be carried through the additional layers of woven electrically-insulating material and electrically-insulating material of the multi-layer skin 308.

In certain embodiments, the second layer 602 is positioned between the first layer 600 and the third layer 604, and the fourth layer 606 is positioned between the third layer 604 and the fifth layer 608. In certain embodiments, the second layer 602 and the fourth layer 606 comprise an electrically-insulating material such as para-aramid fibers.

A multi-layer structure with alternating layers of electrically-conductive and electrically-insulating materials has been found to permit the use of structurally-rigid, lightweight materials such as carbon fiber while mitigating the effects of eddy-current-based magnetic field distortion introduced by carbon fiber. For example, the electrically-insulating material reduces the overall "bulk" volume of the electrically-conductive material. Also, the electrically-insulating material helps interrupt loops of conductive electrically-conductive material that would otherwise exist which, in turn, mitigates the magnitude of generated eddy currents and magnetic fields.

Although five layers are shown in FIG. 6, the multi-layer skin 308 can include fewer or additional layers. For example, a multi-layer skin that is less than .07" thick may comprise eleven different layers (e.g., six layers of woven electrically-insulating material and electrically-insulating material and five layers of electrically-insulating material). In certain embodiments, the layer of electrically-insulating material is thinner than the layer of woven electrically-insulating material and electrically-insulating material.

In certain embodiments, the multi-layer skin 308 is formed by arranging and stacking the various layers (in their fabric state) as discussed above, applying an epoxy to the staked layers of fabric, and then letting the epoxy cure. In certain embodiments, the epoxy is applied to the layers before layers are arranged and stacked. The same epoxy can be used for all of the layers such that the layers are cross-linked. In some embodiments, the multi-layer skin 308 is first formed in sheets and then cut to desired dimensions. The various cut-out parts can be coupled together via non-metallic materials (e.g., epoxies) and/or fasteners to form the exterior shape of the housing 302. In some embodiments, the housing 302 is formed using one or more molds and coupled together using non-metallic materials and/or fasteners. Various components of the housing 302 can be assembled together using non-metallic materials and/or fasteners (e.g., plastic fasteners like clips and screws, epoxies).

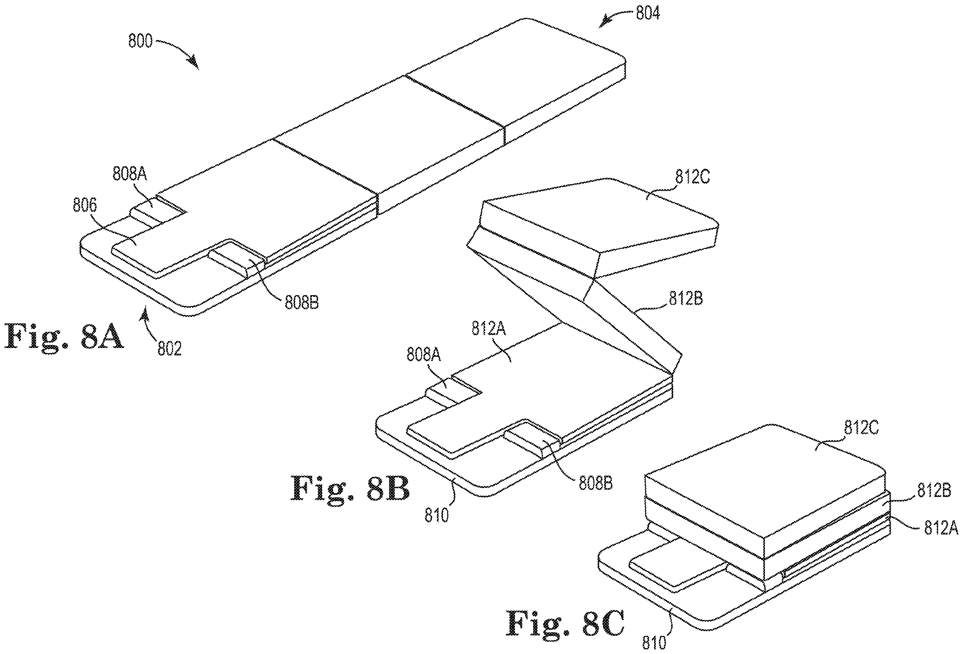

Figures 8A-8C show various arrangements of a magnetic field transmitter assembly 800, which can be used in the tracking system 100 and contain the features described above with respect to the magnetic field transmitter assembly 300 of FIG. 3. The magnetic field transmitter assembly 800 includes a housing 802 and a mattress 804. The housing 802 includes a top surface 806 on which at least part of the mattress 804 rests. The housing 802 also includes a first protruding structure 808A and a second protruding structure 808B. The first protruding structure 808A and the second protruding structure 808B may be integrally formed with the housing 802 (and thus comprising a multi-layer skin) or may be removably coupled to the housing 802. The protruding structures 808A and 808B provide guidance when positioning a patient on the magnetic field transmitter assembly 800. For example, the protruding structures 808A and 808B can be positioned such that a patient's head and/or neck are positioned between the protruding structures 808A and 808B and the top of the patient's shoulders generally align, are positioned below, or rest against the protruding structures 808A and 808B when the patient is resting on the mattress 804.

The mattress 804 can be removable coupled to the housing 802 and able to transition between an extended state (see FIG. 8A) and a folded state (see FIG. 8C). The mattress 804 includes a head-support section 810 and three additional sections (i.e., a first section 812A, a second section 812B, and a third section 812C). The head-support section 810 extends between the protruding structures 808A and 808B and is less wide than the additional sections 812A-C. In certain embodiments, the head-support section 810 and the first section 812A are thinner than the other sections. The other section (i.e., the second section 812B and the third section 812C) can have thickness that is substantially equal to the combined thickness of the housing 802 and the first section 812A such that, in the extended position, the magnetic field transmitter assembly 800 has a substantially uniform thickness. In certain embodiments, a bottom plane 814 along the housing 802 and the mattress 804 is substantially uniform such that the magnetic field transmitter assembly 800 has a flat bottom side that can rests on a flat table surface.

As shown in FIG. 8B, the first section 812A and the second section 812B are separated by a first pivot 816A, and the second section 812B and the third section 812C are separated by a second pivot 816B. The first pivot 816A and the second pivot 816B allow the mattress 804 to be folded into a smaller footprint (e.g., for storage). The pivots 816A and 816B can be a separate component (e.g., pivotable bracket, hook and loop fasteners like Velcro) or an extension of an outer layer of the mattress 804 that can be folded. As shown in FIG. 8C, the second and third sections 812B and 812C of the mattress 804 can be folded such that the second section 812 rests on the first section 812A and a top surface of the protruding structures 808A and 808B while the third section 812C rests on the second section 812B.

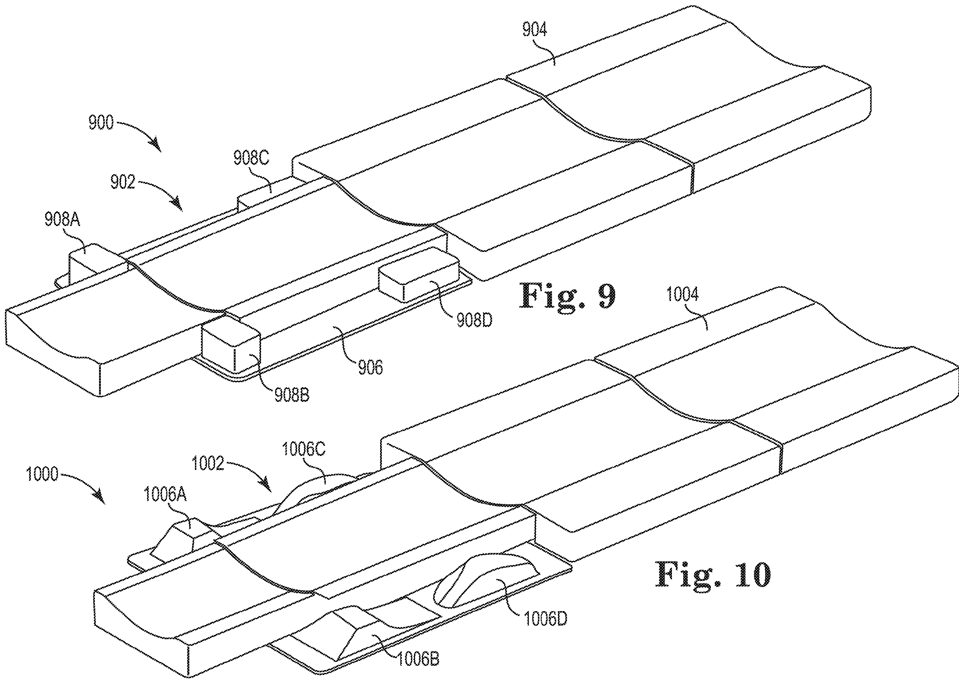

Figures 9-11 show various arrangements of magnetic field transmitter assemblies, each of which can be used in the tracking system 100 and contain the features described above with respect to the magnetic field transmitter assembly 300 of FIG. 3.

FIG. 9 shows a magnetic field transmitter assembly 900 including a housing 902 and a mattress 904. The housing 902 includes a top surface 906 on which at least part of the mattress 904 rests. The housing 902 also includes a first protruding structure 908A, a second protruding structure 908B, a third protruding structure 908C, and a fourth protruding structure 908D. The protruding structures are shown as being cube or cuboid shaped, but other shapes can be used. The protruding structures may be integrally formed with the housing 902 (and thus comprising a multi-layer skin) or may be removably coupled to the housing 902. The protruding structures provide guidance when positioning a patient on the magnetic field transmitter assembly 900. For example, the first and second protruding structures 908A and 908B can be positioned such that a patient's head and/or neck are positioned between the protruding structures, 908A and 908B and the top of the patient's shoulders generally align, are positioned below, or rest against the protruding structures, 908A and 908B, when the patient is resting on the mattress 904. The third and fourth protruding structures, 908C and 908D, are positioned such that a patent's shoulders are positioned between the first and second protruding structures 908A and 908B and the third and fourth protruding structures, 908C and 908D. The mattress 904 is shown in FIG. 9 as being curved to accommodate a patient's body and to help stabilize the patient. The mattress 904 can also include features similar to the mattress 804 of FIG. 8.

FIG. 10 shows a magnetic field transmitter assembly 1000 including a housing 1002 and a mattress 1004. The housing 1002 includes a first protruding structure 1006A, a second protruding structure 1006B, a third protruding structure 1006C, and a fourth protruding structure 1006D. The protruding structures are shown as being shaped to include surfaces that mimic a shape of a body to help provide guidance when positioning a patient on the magnetic field transmitter assembly 1000. For example, the first and second protruding structures 1006A and 1006B include a surface that is curved to accommodate the shape of a patient's shoulder, which would rest against such surfaces. The third and the fourth protruding structures 1006C and 1006D include a surface that is curved to accommodate the shape of a patient's midsection to help maintain a position of the patient. The protruding structures may be integrally formed with the housing 1002 (and thus comprising a multi-layer skin) or may be removably coupled to the housing 1002. The mattress 1004 can include features similar to the mattresses 804, 904 of Figures 8 and 9.

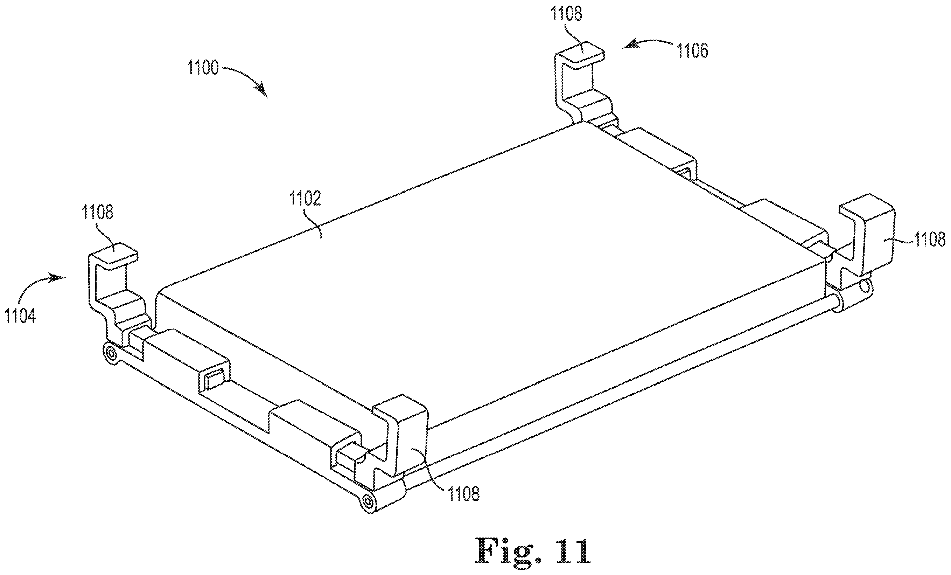

FIG. 11 shows a magnetic field transmitter assembly 1100 including a housing 1102, a first clamp assembly 1104, and a second clamp assembly 1106. Unlike some of the magnetic field transmitter assemblies described above, the magnetic field transmitter assembly 1100 does not have protruding surfaces on the top of the assembly. Using this arrangement, the magnetic field transmitter assembly 1100 can be positioned under a table. For example, the first clamp assembly 1104 and the second clamp assembly 1106 can be used to couple to a table such that the housing 1102 is positioned underneath a table. Each clamp assembly 1104 and 1106 can be adjustable such that clamps 1108 on opposing ends of the assemblies can be positioned (e.g., slid) closer to each other or farther apart from each other to accommodate different-width tables. Further, the clamps 1108 themselves can be adjustable to accommodate different-sized tables. The first and second clamp assemblies 1104 and 1106 can incorporate one or more locking mechanisms to lock the adjustable portions of the assemblies into place once coupled to the table.

## Claims

1. A system comprising:
a magnetic field transmitter assembly (111) including:
a housing including a first layer (600, 604, 608) comprising an electrically-conductive material, a second layer (602, 606) comprising an electrically-insulating material, and a third layer (600, 604, 608) comprising an electrically-conductive material,
wherein the second layer is positioned between the first layer and the third layer, and a plurality of magnetic field generator assemblies positioned within the housing;
wherein the layers comprising an electrically-conductive material (610) further comprise an electrically-insulating material (612), and the layers comprising the electrically- conductive material (610) and the electrically-insulating material (612) are arranged such that the electrically-conductive material and the electrically-insulating material are woven together.

2. The system of claim 1, wherein the plurality of magnetic field generator assemblies includes a coil and/or a permanent magnet.

3. The system of any of claims 1 or 2, wherein each of the plurality of magnetic field generator assemblies includes first and second coil windings in an orthogonal arrangement.

4. The system of any of claims 1-3, wherein each of the plurality of magnetic field generator assemblies includes first, second, and third coil windings in an orthogonal arrangement.

5. The system of any of claims 1-4, wherein each of the plurality of magnetic field generator assemblies is positioned such that respective magnetic fields generated by the plurality of magnetic field generator assemblies overlap each other.

6. The system of any of claims 1-5, wherein coil windings within the plurality of magnetic field generator assemblies are either energized at different periods of time or energized simultaneously at different frequencies from each other.

7. The system of any of claims 1-6, wherein the housing includes a fourth layer comprising an electrically-insulating material and a fifth layer comprising an electrically-conductive material, and wherein the fourth layer is positioned between the third layer and the fifth layer.

8. The system of any of claims 1-7, wherein the layers of the housing form a skin that substantially covers an exterior of the housing.

9. The system of any of claims 1-8, wherein the electrically-conductive material comprises carbon fibers, and wherein the electrically-insulating material comprises para-aramid fibers.

10. The system of any of claims 1-9, wherein the electrically-conductive material comprises a plurality of fibers extending parallel to each other.

11. The system of any of claims 1-10, further comprising: a plurality of reference sensors each positioned adjacent to one of the plurality of magnetic field generator assemblies.

12. The system of any of claims 1-11, further comprising: a magnetic field controller configured to control current applied to the plurality of magnetic field generator assemblies.

13. The system of any of claims 1-12, further comprising: a receiver coupled to a medical device, the receiver configured to sense magnetic fields generated by the plurality of magnetic field generator assemblies and generate one or more sensed field signals; and a signal processor configured to receive the sensed field signals and to determine location of the receiver based on phase of the one or more sensed field signals.

## Patentansprüche

1. System, umfassend:
eine Magnetfeld-Senderbaugruppe (111), umfassend:
ein Gehäuse, umfassend eine erste Schicht (600, 604, 608), die ein elektrisch leitendes Material umfasst, eine zweite Schicht (602, 606), die ein elektrisch isolierendes Material umfasst, und eine dritte Schicht (600, 604, 608), die ein elektrisch leitendes Material umfasst, wobei die zweite Schicht zwischen der ersten Schicht und der dritten Schicht angeordnet ist,
und eine Vielzahl von Magnetfeldgeneratorbaugruppen, die innerhalb des Gehäuses angeordnet sind;
wobei die Schichten, die ein elektrisch leitendes Material (610) umfassen, ferner ein elektrisch isolierendes Material (612) umfassen, und die Schichten, die das elektrisch leitende Material (610) und das elektrisch isolierende Material (612) umfassen, so angeordnet sind, dass das elektrisch leitende Material und das elektrisch isolierende Material miteinander verwoben sind.

2. System nach Anspruch 1, wobei die Vielzahl von Magnetfeldgeneratorbaugruppen eine Spule und/oder einen Permanentmagneten umfasst.

3. System nach einem der Ansprüche 1 oder 2, wobei jede der Vielzahl von Magnetfeldgeneratorbaugruppen erste und zweite Spulenwicklungen in orthogonaler Anordnung enthält.

4. System nach einem der Ansprüche 1 bis 3, wobei jede der Vielzahl von Magnetfeldgeneratorbaugruppen erste, zweite und dritte Spulenwicklungen in orthogonaler Anordnung enthält.

5. System nach einem der Ansprüche 1 bis 4, wobei jede der Vielzahl von Magnetfeldgeneratorbaugruppen so angeordnet ist, dass sich die jeweiligen von der Vielzahl von Magnetfeldgeneratorbaugruppen erzeugten Magnetfelder gegenseitig überlappen.

6. System nach einem der Ansprüche 1 bis 5, wobei die Spulenwicklungen innerhalb der Vielzahl von Magnetfeldgeneratorbaugruppen entweder zu unterschiedlichen Zeitpunkten oder gleichzeitig mit unterschiedlichen Frequenzen erregt werden.

7. System nach einem der Ansprüche 1 bis 6, wobei das Gehäuse eine vierte Schicht mit einem elektrisch isolierenden Material und eine fünfte Schicht mit einem elektrisch leitenden Material aufweist und wobei die vierte Schicht zwischen der dritten Schicht und der fünften Schicht angeordnet ist.

8. System nach einem der Ansprüche 1 bis 7, wobei die Schichten des Gehäuses eine Haut bilden, die im Wesentlichen eine Außenseite des Gehäuses bedeckt.

9. System nach einem der Ansprüche 1-8, wobei das elektrisch leitende Material Kohlenstofffasern umfasst und das elektrisch isolierende Material Para-Aramidfasern umfasst.

10. System nach einem der Ansprüche 1 bis 9, wobei das elektrisch leitende Material eine Vielzahl von parallel zueinander verlaufenden Fasern umfasst.

11. System nach einem der Ansprüche 1 bis 10, ferner umfassend: eine Vielzahl von Referenzsensoren, die jeweils neben einer der Vielzahl von Magnetfeldgeneratorbaugruppen angeordnet sind.

12. System nach einem der Ansprüche 1 bis 11 ferner umfassend: eine Magnetfeldsteuerung, die eingerichtet ist, einen an die Vielzahl von Magnetfeldgeneratorbaugruppen angelegten Strom zu steuern.

13. System nach einem der Ansprüche 1 bis 12, ferner umfassend: einen an ein medizinisches Gerät gekoppelten Empfänger, der eingerichtet ist, von den mehreren Magnetfeldgeneratorbaugruppen erzeugte Magnetfelder zu erfassen und ein oder mehrere erfasste Feldsignale zu erzeugen; und einen Signalprozessor, der eingerichtet ist, die erfassten Feldsignale zu empfangen und den Standort des Empfängers auf der Grundlage der Phase des einen oder der mehreren erfassten Feldsignale zu bestimmen.

## Revendications

1. Système comprenant :
un assemblage d'émetteur de champ magnétique (111) incluant :
un boîtier qui inclut une première couche (600, 604, 608) comprenant un matériau électriquement conducteur, une deuxième couche (602, 606) comprenant un matériau électriquement isolant et une troisième couche (600, 604, 608) comprenant un matériau électriquement conducteur, dans lequel la deuxième couche est positionnée entre la première couche et la troisième couche,
et une pluralité d'assemblages de générateur de champ magnétique qui sont positionnés à l'intérieur du boîtier ;
dans lequel les couches comprenant un matériau électriquement conducteur (610) comprennent en outre un matériau électriquement isolant (612), et les couches comprenant le matériau électriquement conducteur (610) et le matériau électriquement isolant (612) sont agencées de telle sorte que le matériau électriquement conducteur et le matériau électriquement isolant soient entremêlés.

2. Système selon la revendication 1, dans lequel la pluralité d'assemblages de générateur de champ magnétique inclut une bobine et/ou un aimant permanent.

3. Système selon l'une quelconque des revendications 1 ou 2, dans lequel chacun de la pluralité d'assemblages de générateur de champ magnétique inclut des premier et second enroulements de bobine selon un agencement orthogonal.

4. Système selon l'une quelconque des revendications 1 à 3, dans lequel chacun de la pluralité d'assemblages de générateur de champ magnétique inclut des premier, deuxième et troisième enroulements de bobine selon un agencement orthogonal.

5. Système selon l'une quelconque des revendications 1 à 4, dans lequel chacun de la pluralité d'assemblages de générateur de champ magnétique est positionné de telle sorte que des champs magnétiques respectifs qui sont générés par la pluralité d'assemblages de générateur de champ magnétique se chevauchent mutuellement.

6. Système selon l'une quelconque des revendications 1 à 5, dans lequel les enroulements de bobine à l'intérieur de la pluralité d'assemblages de générateur de champ magnétique sont soit mis sous tension à différentes périodes temporelles, soit mis sous tension simultanément à différentes fréquences les uns par rapport aux autres.

7. Système selon l'une quelconque des revendications 1 à 6, dans lequel le boîtier inclut une quatrième couche comprenant un matériau électriquement isolant et une cinquième couche comprenant un matériau électriquement conducteur, dans lequel la quatrième couche est positionnée entre la troisième couche et la cinquième couche.

8. Système selon l'une quelconque des revendications 1 à 7, dans lequel les couches du boîtier forment une peau qui recouvre de façon substantielle un extérieur du boîtier.

9. Système selon l'une quelconque des revendications 1 à 8, dans lequel le matériau électriquement conducteur comprend des fibres de carbone et dans lequel le matériau électriquement isolant comprend des fibres de para-aramide.

10. Système selon l'une quelconque des revendications 1 à 9, dans lequel le matériau électriquement conducteur comprend une pluralité de fibres qui sont étendues parallèlement les unes aux autres.

11. Système selon l'une quelconque des revendications 1 à 10, comprenant en outre : une pluralité de capteurs de référence dont chacun est positionné de telle sorte qu'il soit adjacent à l'un de la pluralité d'assemblages de générateur de champ magnétique.

12. Système selon l'une quelconque des revendications 1 à 11, comprenant en outre : un contrôleur de champ magnétique qui est configuré pour régler un courant qui est appliqué sur la pluralité d'assemblages de générateur de champ magnétique.

13. Système selon l'une quelconque des revendications 1 à 12, comprenant en outre : un récepteur qui est couplé à un dispositif médical, le récepteur étant configuré pour détecter des champs magnétiques qui sont générés par la pluralité d'assemblages de générateur de champ magnétique et pour générer un signal ou plusieurs signaux de champ détecté ; et un processeur de signal configuré pour recevoir les signaux de champ détecté et pour déterminer une localisation du récepteur sur la base de la phase des un ou plusieurs signaux de champ détecté.
